# EUROPÄISCHE PATENTSCHRIFT

(11) **EP 0 588 139 B1**
(45) Veröffentlichungstag und Bekanntmachung des Hinweises auf die Patenterteilung: **21.01.1998**
(21) Anmeldenummer: 93113818.4
(22) Anmeldetag: 30.08.1993
(51) Int. Cl.: C12Q 1/18

(54) **Analytisches Verfahren zur Untersuchung von Gemischen auf toxische Bestandteile**
Analytical method for investigating mixtures for toxic components
Procédé analytique pour la recherche de constituants toxiques dans des mélanges

(30) Priorität: 10.09.1992 DE 4230264
(43) Veröffentlichungstag der Anmeldung: 23.03.1994
(73) Patentinhaber: BAYER AG, 51368 Leverkusen (DE)
(72) Erfinder: Weisemann, Claus, Dr., D-51429 Bergisch Gladbach (DE); Kreiss, Wolfgang, Dr., D-51467 Bergisch Gladbach (DE); Rast, Hans-Georg, Dr., D-51465 Bergisch Gladbach (DE); Eberz, Günther, Dr., D-51375 Leverkusen (DE)

(56) Entgegenhaltungen:
- EP-A- 0 293 775
- WO-A-85/00890
- WO-A-90/04037

## Beschreibung

Zur Toxizitatsprüfung von chemischen Verbindungen werden seit vielen Jahren Leuchtbakterien eingesetzt (siehe z.B. A.A. Bulich; G. Bitton, B.J. Dutkay, "Toxicity Testing Using Microorganisms", CRC Press, Boca-Raton, Florida, USA, Seiten 57 bis 74). Ein solcher Leuchtbakterientest beruht darauf, daß bakterientoxische Substanzen zu einer Reduzierung der durch die Bakterien erzeugten Biolumineszenz führen. Diese Reduzierung wird meßtechnisch ausgewertet. Bei einem Gemisch von verschiedenen chemischen Verbindungen konnte bisher nur die (integrale) Toxizitat des Gesamtgemisches bewertet werden. Über die Toxizität der daran beteiligten Einzelkomponenten konnten keine Aussagen gemacht werden.

Hier setzt die Erfindung an. Es liegt die Aufgabe zugrunde, ein Verfahren zur Untersuchung von Gemischen auf toxische Bestandteile auf der Basis des Leuchtbakterientests zu entwickeln, das eine Zuordnung der gemessenen Toxizitäten zu den Einzelkomponenten des Gemischs ermöglicht. Dabei muß die wichtige Randbedingung erfüllt werden, daß das Meßverfahren selbst keinen schädigenden Einfluß auf die Leuchtbakterien ausübt.

Diese Aufgabe wird erfindungsgemäß dadurch gelöst, daß das zu untersuchende Gemisch zuerst chromatographisch in Fraktionen getrennt wird, daß die getrennten Fraktionen unmittelbar im chromatographischen System mit Leuchtmikroorganismen in Kontakt gebracht werden und die Toxizität der Fraktionen durch Messung der Biolumineszenz bestimmt wird.

Die Trennung der Fraktionen erfolgt vorteilhaft mit Hilfe der Dünnschichtchromatographie oder der Säulenflüssigkeitschromatographie. Im Falle der Dünnschichtchromatographie wird die DC-Platte mit einer Leuchtmikroorganismen-Suspension benetzt und die den einzelnen Fraktionen zugeordnete, lokale Biolumineszenz untersucht.

Zu diesem Zweck wird in vorteilhafter Weise die DC-Platte mit einem photographischen Film in Kontakt gebracht und die den einzelnen Fraktionen zugeordnete Biolumineszenz am belichteten Film visuell oder densitometrisch ausgewertet.

Im Falle der Trennung mit Hilfe der Säulenflüssigkeitschromatographie wird dem Eluat aus der chromatographischen Säule die Leuchtmikroorganismen-Suspension kontinuierlich zugemischt und die Biolumineszenz der Mischung mit Hilfe eines Durchflußphotometers gemessen.

Mit der Erfindung werden folgende Vorteile erzielt:
1. Während bei herkömmlichen Detektionsverfahren in der Chromatographie eine Aussage über die Identität, die Menge und/oder die chemische Struktur einer Gemischkomponente erhalten wird, liefert das erfindungsgemäße Verfahren eine direkte Information zur biologischen Wirkung einer bestimmten Komponente des Gemischs, d.h. die Toxizität einer Mischung kann direkt diskreten Einzelkomponenten dieser Mischung zugeordnet werden. Damit wird eine neuartige Aussagequalität bei der durch chromatographische Trennungen erhältichen Information erreicht.
2. Durch die selektive Toxizitätsdetektion können Aufwand und Kosten bei der Strukturaufklärung unbekannter toxischer Verbindungen in Gemischen erheblich reduziert werden, da die Arbeiten zur Strukturaufklärung (z.B. durch Isolierung und anschließende spektroskopische Untersuchung) auf diejenigen Gemischkomponenten konzentriert werden können, denen aufgrund der Ergebnisse der Toxizitätsdetektion eine toxische Wirkung zugeordnet werden kann. Anwendungsbeispiele hierfür sind die Strukturaufklärung unbekannter toxischer Komponenten in Abwässern oder der Nachweis von Bioziden in verschiedenen Produkten.

Im folgenden wird die Erfindung anhand einer Zeichnung und mit Hilfe von Ausführungsbeispielen näher erläutert.

### 1. Dünnschichtchromatographische Toxizitätsdetektion

Bei der dünnschichtchromatographischen Toxizitätsdetektion mit Leuchtmikroorganismen geht man wie folgt vor:

Die Probe, z.B. ein Extrakt aus einem Produkt, das auf Biozide untersucht werden soll, wird nach laborüblichen Methoden dünnschichtchromatographisch getrennt. Danach kann eine konventionelle Detektion durch Fluoreszenzlöschung erfolgen. Nach vollständigem Abdampfen des Laufmittels wird eine Leuchtmikroorganismen-Suspension(in Wasser mit 3 % Natriumchlorid) durch Aufsprühen oder Eintauchen so auf die DC-Platte aufgebracht, daß ein dünner Film auf der DC-Platte verbleibt. Daraufhin wird die Platte mit einer transparenten Folie oder einer Glasplatte abgedeckt. In einer Dunkelkammer wird nun ein photographischer Film auf diese Glasplatte gelegt und 0,5 bis 15 min lang liegengelassen. Der Film wird danach durch laborübliche Methoden entwickelt und fixiert. Toxische Fraktionen auf der DC-Platte können dann un- oder wenig belichteten Stellen des Films zugeordnet werden.

### Beispiel

- Probe:: Extrakt aus einem Produkt, das auf Biozide untersucht werden soll
- Lösungsmittel:: Methanol / Isopropanol / Wasser (1/1/1 Volumenteile)
- DC-Platte:: Kieselgel 60 mit Fluoreszenzindikator F₂₅₂, Fa. Merck, Darmstadt, Art.-Nr. 5715. Die DC-Platte wird vor der Trennung zweimal mit Methanol gewaschen und anschließend getrocknet
- Laufmittel:: n-Heptan / Dichlormethan / Methanol (15/4/1 Volumenteile). Die Entwicklung erfolgt vertikal in einer einfachen DC-Kammer
- Leuchtmikroorganismen:: Photobacterium phosphoreum
- Film:: Agfa Scopix Video 5B
- Belichtungszeit:: 14 min.

### 2. Hochdruckflüssigkeitschromatographie (HPLC)

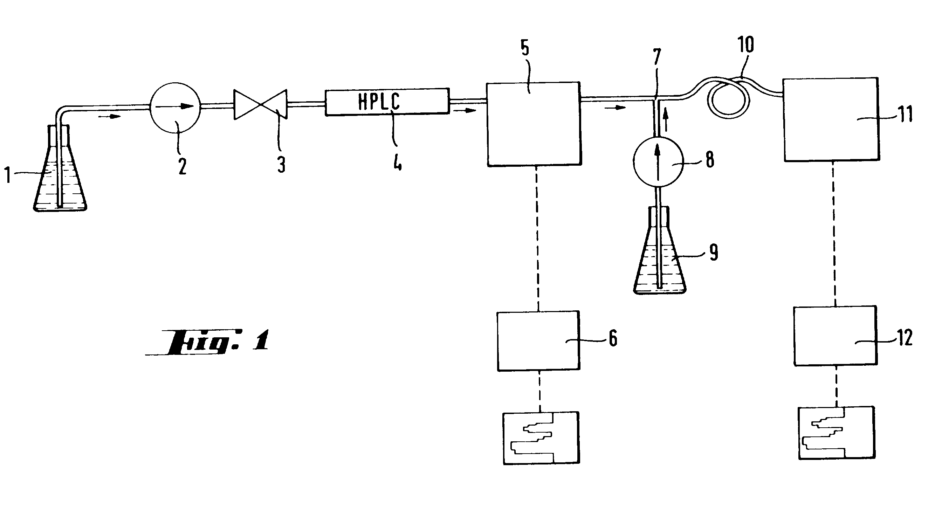

Eine HPLC-Anlage zur Toxizitätsdetektion ist in Fig. 1 schematisch dargestellt. Der Eluent wird aus dem Vorratsgefäß 1 mittels der HPLC-Pumpe 2 über das Probeneingabeventil 3 in die chromatographische Säule 4 eingespeist. Die zeitlich nacheinander aus der Säule 4 austretenden Fraktionen (Eluat) werden in üblicher Weise mit einem UV-Detektor 5 in einer daran angeschlossenen Auswerteeinheit 6 untersucht. Als Meßküvette wird im UV-Detektor 5 eine Durchflußküvette eingesetzt. Dem aus der Durchflußküvette austretenden Eluatstrom wird sodann über ein T-Stück 7 mittels der Dosierpumpe 8 aus dem Vorratsgefäß 9 kontinuierlich eine Leuchtmikroorganismen-Suspension zudosiert. Eluat und Leuchtbakterien-Suspension werden in der Mischstrecke 10 gründlich durchmischt. Die Mischung wird anschließend einem Durchflußphotometer 11 zugeführt, in dem die Biolumineszenz der einzelnen Fraktionen gemessen und ausgewertet wird (Auswerteeinheit 12).

### Beispiel

- Probe:: wäßrige Probe, die auf toxische Komponenten untersucht werden soll
- HPLC-Gerät:: HP 1050 mit Autosampler und MWD-Detektor, Fa. Hewlett-Packard.
- Pumpe zur Förderung der Bakteriensuspension:: BT 8100, Fa. Biotronik
- Datenauswertung:: GINA-Software, Fa. Ravtest
- HPLC-Säule:: Shandon Hypersil ODS, 5 µm; 10 cm x 0,4 cm Durchmesser
- Mischcoil:: biokompatibles Nachsäulen-Derivatisierungssystem, Fa. Biorad
- Durchflußphotometer:: Ramona 90, Fa. Ravtest
- Probenmenge:: 20 µl (Probenschleife)
- Eluent:: Wasser mit 3 % NaCl; 1,5 ml/min.
- Leuchtbakterien-Mengenstrom:: 1 ml/min
- Detektionswellenlänge:: (UV-Detektion): 220 nm
- Bakterien:: Photobakterium phosphoreum, suspendiert in Wasser mit 3 % NaCl, gekühlt auf 0°C.

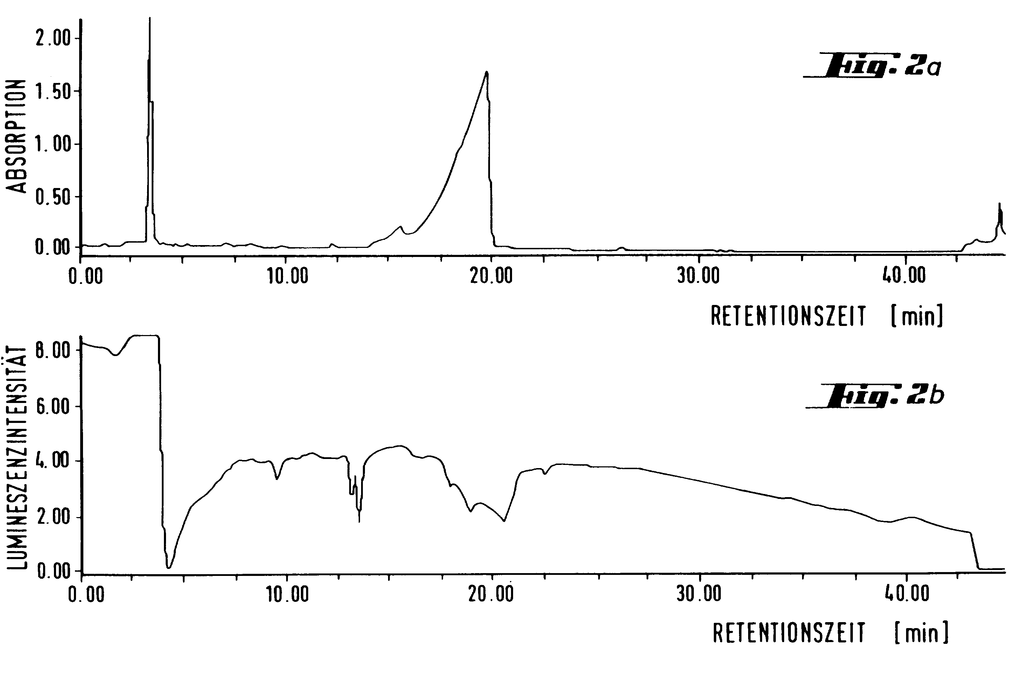

Die registrierten Meßergebnisse sind in Fig. 2a und 2b dargestellt. Die obere Kurve zeigt ein mit dem UV-Detektor 5 und der Auswerteeinheit 6 gemessenes konventionelles HPLC-Chromatogramm einer Probe, die auf toxische Komponenten untersucht werden soll; die untere Kurve zeigt die mit dem Durchflußphotometer 11 und der Auswerteeinheit 12 gemessene Biolumineszenz desselben Eluats.

Man erkennt eine UV-aktive Hauptkomponente bei einer Retentionszeit von ca. 20 min sowie eine Nebenkomponente bei einer Retentionszeit von ca. 15 min.

Bei der Toxizitätsdetektion findet man drei Signalgruppen: Ein kleines Signal bei ca. 10 min, ein Doppelsignal bei ca. 14 min und das zur Hauptkomponente gehörende Signal bei ca. 20 min.

Interessanterweise findet man für die beiden erstgenannten Signalgruppen mit der durchflußphotometrischen Detektion keine Entsprechung. Offensichtlich sind die diese Signale verursachenden Komponenten der untersuchten Mischung mittels UV-Detektion bei 254 nm nicht detektierbar und/oder liegen in nicht ausreichender Konzentration hierfür vor.

Die Lumineszenzabschwächung bei ca. 10 bzw. bei ca. 14 min zeigt jedoch, daß bei diesen Retentionszeiten toxische Komponenten eluiert werden.

Zur Angabe des Ausmaßes der Toxizität kann unter identischen Bedingungen mit der identischen Bakteriensuspension und in zeitlich möglichst geringem Abstand zur Messung der Probe eine definierte Menge einer Substanz bekannter Toxizität chromatographiert werden und die dadurch verursachte Lumineszenzabschwächung wie oben beschrieben gemessen werden. Dieses durch die bekannte Substanz hervorgerufene Toxizitätssignal kann dann als Bezugssignal für durch unbekannte Substanzen hervorgerufene Signale dienen, und man kann eine Angabe darüber machen, um welchen Faktor sich die Größe (angegeben als Peakhöhe oder als Peakfläche) des von der unbekannten Substanz hervorgerufenen und des durch eine bekannte Menge der Vergleichsverbindung hervorgerufenen Signals unterscheiden.

Als besonders geeignet für HPLC-Anwendungen haben sich die Bakterienstämme Vibrio harveyi ATCC 14126 und Xenorhabdus luminescens ATCC 29999 erwiesen. Beide Stämme zeigen überraschenderweise auch bei einer Fließmittelzusammensetzung von bis zu 25 % Methanol in Wasser keine nennenswerte Abnahme der Lumineszenz. Trotzdem werden bakterientoxische Substanzen weiterhin durch eine Abnahme der Lumineszenz detektiert. Damit läßt sich der Anwendungsbereich der HPLC mit Toxizitätsdetektion im Vergleich zur Verwendung von Wasser als Fließmittel weit ausdehnen.

## Patentansprüche

1. Analytisches Verfahren zur Untersuchung von Gemischen auf toxische Bestandteile, dadurch gekennzeichnet, daß das Gemisch zuerst chromatographisch in Fraktionen getrennt wird, daß die getrennten Fraktionen unmittelbar im chromatographischen System mit Leuchtmikroorganismen in Kontakt gebracht werden und die Toxizität der Fraktionen durch Abnahme der Biolumineszenz bestimmt wird.

2. Verfahren nach Anspruch 1, dadurch gekennzeichnet, daß die Trennung der Fraktionen mit Hilfe der Dünnschichtchromatographie erfolgt.

3. Verfahren nach Anspruch 1, dadurch gekennzeichnet, daß die Trennung mit Hilfe der Saulenflüssigkeitschromatographie erfolgt.

4. Verfahren nach Anspruch 2, dadurch gekennzeichnet, daß die Dünnschichtchromatographie-Platte mit einer Leuchtmikroorganismen-Suspension benetzt wird und die den einzelnen Fraktionen zugeordnete, lokale Biolumineszenz untersucht wird.

5. Verfahren nach Anspruch 4, dadurch gekennzeichnet, daß die Dünnschichtchromatographie-Platte mit einem photographischen Film in Kontakt gebracht wird und die den einzelnen Fraktionen zugeordnete Biolumineszenz am belichteten Film visuell oder densitometrisch ausgewertet wird.

6. Verfahren nach Anspruch 3, dadurch gekennzeichnet, daß die Bakterienstämme Vibrio harveyi ATCC 14126 oder Xenorhabdus ATCC 29999 als Leuchtmikroorganismen verwendet werden.

## Claims

1. Analytical process for assaying mixtures for toxic constituents, characterised in that the mixture is first separated into fractions by chromatography, the separated fractions are brought into contact with luminescent microorganisms within the chromatographic system itself and the toxicity of the fractions is determined by the reduction in bioluminescence.

2. Process according to claim 1, characterised in that the fractions are separated with the assistance of thin layer chromatography.

3. Process according to claim 1, characterised in that separation proceeds with the assistance of column liquid chromatography.

4. Process according to claim 2, characterised in that the thin layer chromatography plate is wetted with a suspension of luminescent microorganisms and the local bioluminescence associated with the individual fractions is assayed.

5. Process according to claim 4, characterised in that the thin layer chromatography plate is brought into contact with a photographic film and the bioluminescence associated with the individual fractions is evaluated on the exposed film visually or densitometrically.

6. Process according to claim 3, characterised in that the bacterial strains *Vibrio harveyi* ATCC 14126 or *Xenorhabdus* ATCC 29999 are used as the luminescent microorganisms.

## Revendications

1. Procédé analytique de détection des composants toxiques de mélanges, caractérisé en ce que le mélange est d'abord séparé en fractions par chromatographie, que les fractions séparées sont immédiatement mises en contact avec des micro-organismes luminescents dans le système de chromatographie et que la toxicité des fractions est déterminée par la diminution de la bioluminescence.

2. Procédé selon la revendication 1, caractérisé en ce que la séparation des fractions s'effectue par chromatographie en couche mince.

3. Procédé selon la revendication 1, caractérisé en ce que la séparation s'effectue par chromatographie en phase liquide sur colonne.

4. Procédé selon la revendication 2, caractérisé en ce que la plaque de chromatographie en couche mince est mouillée par une suspension de micro-organismes luminescents et que la bioluminescence locale, imputée aux différentes fractions, est détectée.

5. Procédé selon la revendication 4, caractérisé en ce que la plaque de chromatographie en couche mince est mise en contact avec un film photographique et que la bioluminescence imputée aux differentes fractions est évaluée visuellement ou par densitométrie sur le film exposé.

6. Procédé selon la revendication 3, caractérisé en ce qu'on utilise les souches bactériennes Vibrio harveyi ATCC 14126 ou Xenorhabdus ATCC 29999 comme micro-organismes luminescents.
